# FASCICULE DE BREVET EUROPEEN

(11) **EP 2 676 662 B1**
(45) Date de publication et mention de la délivrance du brevet: **29.03.2017**
(21) Numéro de dépôt: 12305691.3
(22) Date de dépôt: 18.06.2012
(51) Int. Cl.: A61K 9/70, A61L 15/18, D01F 1/10, D01F 6/60, A61F 13/00, A61P 43/00, A61B 18/04

(54) **Composition polymérique contenant des charges minérales, pour améliorer la cicatrisation de la peau**
Polymerzusammensetzung mit mineralischen Füllstoffen zur Verbesserung der Narbenbildung der Haut
Polymeric composition containing mineral fillers, for improving skin wound healing

(43) Date de publication de la demande: 25.12.2013
(73) Titulaire: Rhodia Poliamida E Especialidades Ltda, Sao Paulo - SP (BR)
(72) Inventeur: Cordeiro Bastos, Tarcis, 04014-011 Sao Paulo (BR); Gorescu, Gabriel, 09290-520 Sao Paulo (BR); Canova, Thomas Gonzaga, Sao Paulo (BR)
(74) Mandataire: Casalonga

(56) Documents cités:
- WO-A1-94/29499
- WO-A1-2012/069902
- WO-A2-2009/077834
- FR-A1- 2 756 742
- FR-A1- 2 908 313
- US-A1- 2010 292 624
- WIN-CHUN JAO ET AL: "Fabrication and characterization of electrospun silk fibroin/TiO2 nanofibrous mats for wound dressings", POLYMERS FOR ADVANCED TECHNOLOGIES, vol. 23, no. 7, 27 août 2011 (2011-08-27), pages 1066-1076, XP055030730, ISSN: 1042-7147, DOI: 10.1002/pat.2014
- "Sell tourmaline shoulder brace", ec21 , 31 mars 2012 (2012-03-31), XP002687794, Extrait de l'Internet: URL:http://www.ec21.com/offer_detail/Sell_ tourmaline_shoulder_brace--12835405.html [extrait le 2012-11-21]
- TOYOKAWA HIDEYOSHI ET AL: "Promotive effects of far-infrared ray on full-thickness skin wound healing in rats", EXPERIMENTAL BIOLOGY AND MEDICINE (MAYWOOD), [Online] vol. 228, no. 6, juin 2003 (2003-06), pages 724-729, XP002687795, ISSN: 1535-3702 Extrait de l'Internet: URL:http://ebm.rsmjournals.com/content/228 /6/724.full.pdf> [extrait le 2012-11-21]

## Description

L'invention est définie dans les revendications annexées. Toute divulgation allant au-delà de la portée desdites revendications est uniquement destinée à des fins d'illustration.

La présente invention a pour objet une composition polymérique ainsi que des fils, fibres ou filaments, des films, des articles textiles et des dispositifs médicaux obtenus à partir de cette composition, utiles pour améliorer la cicatrisation de la peau, notamment à travers la stimulation du fonctionnement des kératinocytes et des fibroblastes, entre autres en activant la synthèse du collagène à la surface de la peau lésée.

La peau humaine est constituée de trois tissus superposés: l'épiderme, le plus externe, le derme, et l'hypoderme, le plus profond.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier car il contient une vascularisation dont l'épiderme est exempt. II est principalement constitué de fibroblastes et d'une matrice extracellulaire composée elle-même de différentes protéines extracellulaires parmi lesquelles figurent notamment les fibres de collagène, l'élastine et différentes glycoprotéines.

L'hypoderme, qui s'invagine dans le derme et est rattaché au derme sus-jacent par des fibres de collagène et d'élastine, est essentiellement constitué d'un type de cellules spécialisées dans l'accumulation et le stockage des graisses, les adipocytes. Il est le réservoir énergétique de l'organisme.

Les adipocytes sont des cellules matures issues d'un processus de différenciation des fibroblastes en pré-adipocytes et adipocytes; ce processus est nommé adipogénèse et/ou différenciation adipocytaire.

Le collagène est la principale protéine de la matrice extracellulaire (ECM) et est la protéine la plus abondante chez les mammifères, comprenant 25% des protéines totales et 70% à 80% de la peau (poids sec). Le collagène agit comme un échafaud (« scaffold ») structurel dans les tissus. La principale caractéristique de toutes les molécules de collagène est leur rigidité due à la structure à trois brins hélicoïdaux. Les types I, II et III du collagène sont les principaux types de collagène présents dans les tissus conjonctifs et constituent 90% de tout le collagène du corps.

La biosynthèse du collagène commence dans le réticulum endoplasmique, où des chaînes polypeptidiques sont synthétisées. Ces polypeptides passent à travers le réticulum endoplasmique des fibroblastes qui synthétisent les résidus de proline et de lysine qui sont hydroxylés et glycosylés. Il s'ensuit la conformation des polypeptides sous forme de triple hélice, appelé le « procollagène ». Les molécules de procollagène passent ensuite par l'appareil de Golgi où elles sont encapsulées dans des granules sécrétoires et sécrétées vers l'espace extracellulaire dans le tissu conjonctif. Alors, après avoir été excrété, le procollagène, sous l'action d'enzymes extracellulaires, subit le clivage des domaines non hélicoïdaux, ce qui aboutit à la molécule de collagène. Les molécules de collagène s'organisent alors spontanément en fibrilles. Enfin, après l'action de l'enzyme lysyoxydase extracellulaire, les fibrilles forment spontanément le réticule.

La peau peut subir des lésions ou plaies. La lésion peut être de plusieurs types, il s'agit par exemple du résultat d'un traumatisme mécanique (coupure, contusion, lacération, éraflure, morsure), thermique (brûlure), électrique (électrocution) ou chimique (brûlure).

Il peut aussi s'agir de plaies causées par exemples par des ulcères veineux (chez les personnes diabétiques par exemple, ou post-thrombotiques ou liés à une insuffisance veineuse chronique) ou des escarres.

Le collagène est une composante clé dans la guérison des plaies, qui est un processus complexe, qui peut se diviser en 5 phases :

### 1. Coagulation sanguine (Hémostasie)

Les vaisseaux sanguins sont les structures les plus susceptibles d'être endommagées en cas de blessure. Le premier objectif du processus de réparation du corps consiste à arrêter le saignement. L'agrégation plaquettaire et l'activation de la cascade de coagulation causent la coagulation du sang. Les plaquettes sanguines libèrent des aA-granulés, qui libèrent plusieurs facteurs de croissance (FG) et de cytokines, qui "attirent" une variété de cellules inflammatoires (neutrophiles, éosinophiles et monocytes) vers le site de la plaie et lancent la phase inflammatoire.

### 2. Exsudation /phase inflammatoire

Durant cette phase, la dilatation des vaisseaux situés près de la plaie entraîne un écoulement séreux, qui cause un oedème de la plaie. Ce liquide, appelé l'exsudat, contient une variété de substances essentielles comme des enzymes, des anticorps et des cellules inflammatoires, qui sont toutes nécessaires au processus de guérison. Les cellules inflammatoires sécrètent les enzymes protéolytiques notamment les neutrophiles, les éosinophiles et les macrophages. L'action d'enzymes protéolytiques sur les constituants macromoléculaires de l'ECM (tels que le collagène) donne lieu à de nombreux peptides (fragments de protéines) au cours de la cicatrisation des plaies. Ces produits de dégradation ont un effet chimiotactique dans le recrutement d'autres cellules, telles que les cellules mononucléées, les neutrophiles supplémentaires, et les macrophages. Les macrophages activés sécrètent le TNF- a, qui, entre autres, induit les macrophages à produire de l'IL-1bβ. Ces composés (TNF-α et d'IL-1bβ) sont la clé des cytokines pro-inflammatoires qui influent directement sur la formation de collagène dans la plaie en stimulant la synthèse de collagène par les fibroblastes. Les cellules inflammatoires sécrètent également des facteurs de croissance qui continuent à stimuler la migration des fibroblastes, des cellules épithéliales et des cellules endothéliales vasculaires dans la plaie. En conséquence, la cellularité de la plaie augmente.

### 3. Phase de granulation

La régénération des nouvelles cellules - afin de remplacer le tissu endommagé - prédomine durant la phase de guérison suivante. Le nouveau tissu est appelé tissu de granulation. Il remplit la plaie par le dessous et revêt une apparence rouge vif. La construction du nouveau tissu est exécutée par les fibroblastes (les cellules principales du derme) grâce à la synthèse des fibres de collagène qui forment la matrice du tissu conjonctif. Durant cette phase il y a des fibroblastes, des cellules endothéliales vasculaires et aussi des kératinocytes. Les cellules endothéliales vasculaires sécrètent une variété de FG qui favorisent l'angiogénèse. La granulation est atteinte par la vascularisation de l'ECM.

### 4. Phase de ré-épithélialisation

Alors que les nouveaux vaisseaux sont responsables du transport des substances nutritives vers la zone régénérée, le tissu de granulation remplit la plaie et crée la base de la ré-épithélialisation : la construction d'une nouvelle couche de peau. La ré-épithélialisation de la plaie complète le processus de guérison. Les cellules épithéliales se divisent et migrent à partir des bords de la plaie et la referment. Une fois que l'épithélium sous croûte est renouvelé, la croûte se détache et le nouveau tissu épithélial rosé situé en dessous devient visible.

### 5. Phase de maturation

La phase de ré-épithélialisation de la plaie est suivie par la phase de maturation durant laquelle les fibres de collagène sont réorganisées pour donner plus de résistance à la peau. Toutefois, le nouveau tissu n'est pas identique au tissu original, il est parfois inégal et moins élastique. En outre, des variations de couleur importantes sont possibles et dans jusqu'à 15 % des blessures, on peut observer la formation d'une cicatrice hypertrophique. Le processus de remodelage du tissu cicatriciel peut durer des années.

Afin de protéger les plaies des éléments extérieurs pendant la cicatrisation, il est connu d'utiliser des pansements.

Les types de pansements existants sont nombreux et varient selon la nature des plaies. Une des formes les plus communes du pansement est une fine compresse maintenue sur la plaie par un adhésif. Le film adhésif semi-perméable permet de laisser passer l'air et la vapeur d'eau. Il est très souple et peut être utilisé au niveau d'articulations par exemple. Il peut être associé à une couche de gel d'hydrocolloïdes, d'alginates ou à un hydrogel, permettant une meilleure hydratation de la plaie et une cicatrisation favorisée. Le gel peut être imprégné de certains produits tels que des antiseptiques ou des corticoïdes pour limiter les infections ou l'inflammation de la plaie.

Les pansements mettent en oeuvre des fibres, par exemple sous la forme de textile.

Une grande variété de fibres peuvent être utilisées pour la production de matières textiles et ce pour diverses d'applications comme par exemple l'habillement.

Pour la production de matières textiles, les fibres doivent présenter certaines propriétés comme par exemple la ténacité, l'élasticité, la filabilité, etc. Cependant, toutes les fibres ne sont pas adaptées à la fabrication de produits à usage médical.

En effet, les fibres à usage médical doivent présenter également des propriétés telles que la non-toxicité, être stérilisables, avoir une bonne biocompatibilité, la biodégradabilité, une bonne absorbabilité, et avoir un touché doux. Par « textiles à usage médical », on entend les dispositifs non implantables, implantables, extracorporels, les produits de santé et les produits d'hygiène. Les pansements sont considérés comme étant des dispositifs non implantables.

Malgré les nombreux produits actuellement présents sur le marché existe toujours un besoin de proposer de nouvelles solutions pour favoriser la cicatrisation des plaies.

Un des buts de la présente invention est donc de proposer un nouveau produit très efficace, permettant d'améliorer le processus de cicatrisation des plaies.

Dans le domaine cosmétique, la demande WO 2009/077834 décrit des articles textiles à base de polymères contenant des additifs ayant des propriétés d'émission et/ou d'absorption dans la région de l'infrarouge, qui permettent notamment de favoriser l'élimination de la cellulite. Ces additifs sont incorporés dans des compositions polymériques, notamment à base de polyamide, qui sont ensuite filées pour constituer des fibres dites « actives » utilisables par exemple pour concevoir des articles textiles permettant de diminuer la cellulite.

WIN-CHUN JAO ET AL ("Fabrication and characterization of electrospun silk fibroin/TiO2 nanofibrous mats for wound dressings", POLYMERS FOR ADVANCED TECHNOLOGIES, vol. 23, no. 7, 27 août 2011 (2011-08-27), pages 1066-1076, le textile de tourmaline ("Sell tourmaline shoulder brace", ec21, 31 mars 2012 (2012-03-31), Extrait de l'Internet:URL:http://www.ec21.com/offer_detail/ Sell_tourmaline_shoulder_brace--12835405.html, [extrait le 2012-11-21]), US 2010/292624, WO 94/29499, FR 2 908 313, FR 2 756 742, WO 2012/069902 décrivent des matériaux comprenant une charge minérale.

Après de longues recherches, les inventeurs ont découvert qu'une composition polymérique particulière, contenant une dispersion de charges minérales qui présentent une capacité d'émission et/ou absorption de radiations infrarouges dans la plage de longueur d'onde située entre 2 µm et 20 µm permet d'améliorer la cicatrisation de la peau, notamment via l'activation de la synthèse du collagène.

La composition selon l'invention permet également d'augmenter la prolifération des fibroblastes et des kératinocytes, ainsi que leur migration.

En particulier, la composition selon l'invention permet d'améliorer tant le processus (notamment la vitesse et l'efficacité) de la cicatrisation que la qualité de celle-ci.

Plus précisément, l'invention concerne une composition polymérique telle que définie dans la revendication 1
comprenant une matrice polymérique et des charges
minérales qui sont uniformément dispersées dans la matrice polymérique et ont des propriétés d'absorption et/ou d'émission dans la région d'infrarouge lointain allant de 2 µm à 20 µm, pour son utilisation pour améliorer la cicatrisation de la peau.

La composition selon l'invention peut avantageusement se présenter sous forme de fils, fibres ou filaments, lesquels peuvent être en particulier contenus dans un article textile, tel que par exemple un tissu, un tricot ou un non-tissé.

La composition selon l'invention peut également se présenter sous forme de film.

L'invention met en oeuvre une composition comprenant une matrice polymérique. cellulose-ester tels que l'acétate de cellulose, le propionate de cellulose, le rayon, la viscose et les polymères de la même famille, les polymères et copolymères acryliques, les polyamides, le polyhexaméthylène adipamide (PA66) ou le polycaproamide (PA6), ou leurs copolymères en toutes proportions, ou encore des mélanges entre n'importe quels polymères cités précédemment.

Selon une forme préférentielle de réalisation, la matrice polymérique est à base de polyamide, choisi entre le polyamide 6, le polyamide 66 et les copolymères de polyamide 6/polyamide 66 en toutes proportions.

La composition selon l'invention comprend des charges minérales ayant des propriétés d'absorption et/ou d'émission dans la région d'infrarouge lointain allant de 2 à 20 µm. De préférence, les charges minérales ont des propriétés d'absorption et/ou d'émission dans la région d'infrarouge lointain allant de 3 à 20 µm, et encore plus préférentiellement de 3 à 15 µm.

Selon l'invention, les charges minérales sont dispersées uniformément dans la matrice polymérique. Par « uniformément dispersées » on entend que les charges minérales sont incorporées de manière homogène au sein même du polymère. En particulier, les particules sont piégées dans la composition de polymère. Il ne s'agit donc pas de charges minérales déposées sur le polymère, par exemple sous la forme d'un revêtement à la surface du polymère (« coating »).

Une telle dispersion uniforme peut être obtenue en incorporant la ou les charges minérales dans le polymère lors de la synthèse de ce dernier. Un mode de réalisation consiste à réaliser une ou plusieurs suspensions de charges minérales, stabilisée(s) par des tensioactifs. La (les) suspension(s) est(sont) ensuite ajoutée(s) au cours de la synthèse du polymère.

L'on peut également incorporer lesdites charges par mélange de celles-ci au polymère fondu, soit directement, soit au moyen d'un concentré de particules sous forme de mélange maître (« masterbatch »), celui-ci pouvant être postérieurement dilué en concentrations prédéterminées dans la masse polymérique. Cette incorporation dans le polymère fondu peut être avantageusement effectuée au moment de la mise en forme de la composition polymérique, par exemple au moment de l'extrusion de la composition polymérique.

Grâce à de tels procédés l'on peut obtenir des compositions de polymère selon l'invention qui contiennent la ou les charges minérales de façon uniformément dispersée dans la matrice polymère.

Selon l'invention, la composition comprend des charges minérales ayant des propriétés d'absorption et/ou d'émission dans la région d'infrarouge lointain allant de 2 à 20 µm, c'est-à-dire qu'au moins trois types de charges minérales ayant de telles propriétés sont présentes dans ladite composition.

Les charges minérales sont choisies parmi les oxydes, les sulfates, les carbonates, les phosphates et les silicates.

De préférence, l'oxyde est choisi parmi le dioxyde de titane, le dioxyde de silicium et l'oxyde de magnésium.

Le sulfate peut être choisi parmi les sulfates de métaux alcalins et de métaux alcalino-terreux, de préférence parmi le sulfate de baryum, le sulfate de calcium et le sulfate de strontium.

Le carbonate est choisi avantageusement parmi le carbonate de calcium ou de sodium.

De préférence, le silicate est choisi parmi l'actinolite, la tourmaline, la serpentine et le kaolin.

Le phosphate peut être choisi parmi les phosphates de zirconium, l'apatite ou leurs mélanges.

Selon un mode de réalisation avantageux de l'invention, les charges minérales sont choisies parmi les oxydes, sulfates, carbonates, phosphates et silicates.

La composition de
l'invention contient
au moins trois charges minérales de types
différents, choisies parmi les types suivants : les oxydes, les sulfates, et les silicates.

D'autres charges peuvent êtres des carbonates, phosphates.

Selon un mode de réalisation de l'invention, la composition de l'invention contient trois charges minérales différentes, les trois charges étant un oxyde, un sulfate, et un silicate.

Selon un mode de réalisation avantageux de l'invention, la composition polymérique contient au moins trois charges minérales choisies parmi le dioxyde de titane, un sulfate de métal alcalin ou alcalino-terreux et un silicate, et de préférence parmi le dioxyde de titane, le sulfate de baryum, et la tourmaline.

De façon particulièrement préférée, la composition de l'invention comprend trois charges minérales de types différents. L'association des trois charges minérales est de préférence l'association dioxyde de titane/sulfate de métal alcalino-terreux/silicate ; de préférence l'association dioxyde de titane/ sulfate de baryum/ tourmaline.

Dans ce cas, la proportion en poids des trois charges minérales est de préférence comprise entre 80:10:10 et 10:30:60, et plus spécifiquement en proportion de 50:25:25.

Selon un mode de réalisation de l'invention, la proportion en poids de l'association de charges minérales par rapport au poids total de la composition polymérique (matrice + charges) est supérieure ou égale à 1,0 %, de préférence supérieure ou égale à 1,5 % et plus préférentiellement encore supérieure ou égale a 2,5 %.

De préférence, la proportion en poids de l'association de charges minérales par rapport au poids total de la composition polymérique est inférieure ou égale à 9%, de préférence inférieure ou égale à 6 %, avantageusement inférieure ou égale à 4, 5%.

Les charges minérales selon l'invention se présentent sous forme de particules, lesquelles présentent avantageusement une taille moyenne en volume inférieure ou égale à 2 µm, mesurée selon la méthode d'analyse granulométrique par diffraction laser (en utilisant, par exemple, des granulomètres MALVERN ou CILAS).

Une manière avantageuse de procéder consiste à mettre les particules en suspension dans l'eau, et à déterminer leur granulométrie par diffraction laser en employant la méthode décrite dans la norme ISO 13320 :2009.

Il est préférable que les charges minérales utilisées dans la présente invention présentent une taille de particules qui ne soit ni trop petite, pour prévenir tout risque que les particules ne puissent sortir de la matrice polymérique et, s'introduire dans les plaies et pénétrer dans le corps humain ou se disperser dans l'environnement, ni trop grosse, ce qui rendrait plus difficile l'incorporation des particules dans la matrice polymérique et rendrait le matériau plus abrasif au contact de la peau, ce qui pourrait s'avérer nuisible au processus de cicatrisation.

Ainsi, les charges minérales selon l'invention se présentent sous forme de particules qui présentent avantageusement une taille moyenne en volume, mesurée selon la méthode d'analyse granulométrique par diffraction laser, allant de 0,1 à 2 µm, et plus préférentiellement de 0,2 à 1,5 µm.

Les charges minérales présentent avantageusement une distribution granulométrique avec 99 % en volume des particules ayant une taille inférieure à 1,0 µm, de préférence 90 % en volume des particules ayant une taille inférieure à 0,5 µm. La distribution granulométrique est également mesurée par la méthode d'analyse granulométrique par diffraction laser (en utilisant, par exemple, des granulomètres MALVERN ou CILAS).

Lorsque la composition selon l'invention se présente sous forme de fils, fibres ou filaments, le ratio entre la taille de particules des charges minérales et le diamètre du filament est avantageusement optimisé pour éviter les problèmes décrits ci-avant.

Ainsi, le ratio entre le diamètre moyen équivalent des filaments selon l'invention et la taille moyenne en volume des charges minérales, mesurée selon la méthode d'analyse granulométrique par diffraction laser, est avantageusement supérieur ou égal à 10. Ce ratio entre le diamètre moyen équivalent des filaments et la taille moyenne en volume des charges minérales est de préférence inférieur ou égal à 200.

Le diamètre moyen équivalent des filaments est avantageusement mesuré par microscopie optique.

Les fils, fibres ou filaments selon l'invention sont caractérisés par le fait que les filaments présentent de préférence une masse linéaire (ou titre) allant de 0,2 à 20 dtex, avantageusement de 0,5 à 8 dtex, et encore plus préférentiellement de 0,5 à 3,5 dtex.

Le titre des filaments peut varier selon l'application choisie. En effet, pour une application du type « pansement », les filaments présentent avantageusement un titre allant de 0,5 à 1,5 dtex, alors que pour des applications pour des vêtements de compression du type « postopératoires », les filaments peuvent présenter un titre allant de 2 à 8 dtex.

Les filaments selon l'invention présentent de préférence un diamètre moyen équivalent allant de 4 à 50 µm, de préférence de 6 à 30 µm.

Ainsi, de manière particulièrement préférée, lorsque le diamètre moyen du filament va de 4 à 20 µm les charges minérales se présentent sous forme de particules présentant une taille moyenne en volume, mesurée selon la méthode d'analyse granulométrique par diffraction laser, allant de 0,1 µm à 0,4 µm, et plus préférentiellement de 0,2 µm à 0,4 µm.

Aussi, lorsque le diamètre moyen du filament va de 20 à 50 µm les charges minérales se présentent avantageusement sous forme de particules présentant une taille moyenne en volume, mesurée selon la méthode d'analyse granulométrique par diffraction laser, allant de 0,25 µm à 2 µm, et plus préférentiellement de 1 à 2 µm.

La composition selon l'invention peut également se trouver sous forme de film. Les films selon l'invention peuvent être préparés par des procédés classiques, par exemple par soufflage ou coulage sous forme de feuille mince de la composition polymérique. Il est possible d'employer des dispositifs d'extrusion classiques, et de mettre en oeuvre tous post-traitements appropriés (par exemple humidification, recuit).

Comme expliqué plus haut, les charges minérales peuvent être incorporées dans la matrice polymérique au cours de la phase de synthèse du polymère, ou au cours de sa phase de mise en forme, par mélange soit direct soit au moyen d'un concentré de particules sous forme de mélange maître (« masterbatch »), celui-ci pouvant être postérieurement dilué en concentrations prédéterminées dans la masse polymérique au cours de la phase de mise en forme.

La composition polymérique selon l'invention présente de préférence un nombre de pics d'absorption de radiations infrarouges supérieur à 10 dans les dix plages de fréquence suivantes : 3,00 +/-0,30µm, 6,20 +/- 0,50µm, 8,00 +/- 0,25µm, 8,50 +/- 0,25µm, 9,00 +/-0,25µm, 9,50 0,25µm, 10,00 +/- 0,25µm, 10,50 +/- 0,25µm, 11,00 +/- 0,25µm, 14,60 +/- 2,10µm, au moins 1 pic étant présent dans au moins 7 de ces dix plages de fréquence.

Le spectre d'absorption de radiations infrarouges peut être déterminé par toute méthode connue de l'homme du métier. Une méthode possible est l'utilisation d'un appareil Bruker Equinox 55, avec une résolution de 4 cm⁻¹. Dans ce cas le spectre obtenu est sous forme ATR (« Atenuated Total Reflectance »), en utilisant un cristal ZnSe.

La composition selon l'invention peut avantageusement présenter des fonctionnalités supplémentaires, différentes de la fonctionnalité d'émission/absorption dans l'infrarouge lointain (FIR). Il peut s'agir notamment d'une ou plusieurs des fonctionnalités ci-dessous :
- régulation de l'humidité,
- protection contre les microbes,
- hydrophobie/hydrophilie
- capacité d'absorption/capillarité,
- anti-odeurs,
- antifongique,
- insectifuge,
- protection contre les UV,
- antitaches.

Ces fonctionnalités supplémentaires peuvent être apportées par des additifs/actifs, ajoutés à la composition selon l'invention.

Le procédé pour obtenir des fibres selon l'invention peut consister à réaliser une ou plusieurs suspensions de charges minérales comme par exemple un silicate, le dioxyde de titane et un sulfate de métal alcalin ou alcalino-terreux, stabilisée(s) par des tensioactifs. La(les) suspension(s) est(sont) ensuite ajoutée(s) à la synthèse du polyamide. Une alternative est d'introduire une partie des charges minérales, préalablement mis sous la forme d'un masterbatch dans le polymère fondu au moment du filage. Le polyamide obtenu est refroidi, coupé et refondu avant de passer au travers d'une extrudeuse pour former le fil.

Grâce à ce procédé par exemple, les fils, fibres ou filaments en polyamide selon l'invention sont constitués d'une composition polymérique contenant les charges minérales de façon uniformément dispersée dans la matrice polymère.

Dans le cas de fibres obtenues par filage à l'état fondu, les charges minérales peuvent être introduites dans le polymère fondu au moyen d'un dispositif de mélange, par exemple en amont d'un dispositif de filage. Par le filage de la composition de polymère additivée on peut obtenir des fils multifilamentaires continus, des monofilaments, des fibres courtes et longues, ou leurs mélanges. On appellera « fils » l'ensemble des fils, fibres et filaments qui peuvent être obtenus par filage. Les fils obtenus à partir des compositions polymériques chargées présentées plus haut peuvent être soumis à tous les traitements textiles connus de l'homme du métier, tels qu'extrusion, étirage, texturation, teinture, finition etc.

Comme exposé ci-avant, les fils, fibres ou filaments obtenus à partir de la composition selon l'invention permettent notamment de préparer des articles textiles, qui sont efficaces pour améliorer la cicatrisation de la peau.

De tels articles textiles peuvent être obtenus à partir d'un seul type de fils, fibres ou filaments constitués de la composition selon l'invention, ou à partir d'un mélange de fils, fibres ou filaments constitués de la composition selon l'invention avec des fils, fibres ou filaments différents de ceux de l'invention. Les fils, fibres ou filaments de différents de ceux de l'invention peuvent avantageusement présenter des fonctionnalités différentes et/ou complémentaires de la fonctionnalité d'émission/absorption dans l'infrarouge lointain (FIR). Il peut s'agir notamment de fils, fibres ou filaments avec une ou plusieurs des fonctionnalités ci-dessous :
- régulation de l'humidité,
- protection antimicrobienne,
- hydrophobie ou hydrophilie,
- capacité d'absorption d'eau /capillarité,
- anti-odeurs,
- antifongique,
- insectifuge,
- protection contre les UV,
- anti-adhésive.

Ces fonctionnalités peuvent être apportées par des additifs/actifs, ajoutés aux fils, fibres ou filaments différents de ceux de l'invention lors de leur préparation.

Par « article textile », on entend notamment un tissu, un tricot ou un non-tissé.

L'article textile est fabriqué par des techniques connues en utilisant les fils, fibres ou filaments constitués de la composition de l'invention comme matière première, et éventuellement d'autres fils, fibres ou filaments naturels (par exemple du coton) ou de synthèse (par exemple la viscose). Ces fils, fibres ou filaments additionnels peuvent notamment avoir une bonne hygroscopicité, ce qui peut être avantageux dans l'application.

Selon un mode de réalisation particulièrement avantageux, l'article textile se présente sous la forme d'un bandage ou d'un vêtement, comme par exemple un bermuda, un t-shirt, un collant, un pantalon, un bas de contention ou un vêtement de compression postopératoire (du type de ceux utilisés en chirurgie classique ou esthétique).

Quelle que soit sa forme, la composition selon l'invention peut être contenue dans un dispositif médical non implantable.

Notamment, les fils, fibres ou filaments (éventuellement incorporés dans un article textile), les films préparés à partir de la composition selon l'invention permettent de préparer des dispositifs médicaux non implantables, qui sont utiles pour améliorer la cicatrisation de la peau.

Le dispositif médical non implantable est notamment un pansement ou un fil de suture. Le pansement peut être une gaze, un pansement adhésif, un emplâtre, un bandage tel qu'un bandage de compression ou un bandage absorbant, un ruban adhésif ou un support tissulaire « scaffold ».

Le dispositif médical non implantable selon l'invention peut par exemple comprendre les fils, fibres ou filaments obtenus à partir de la composition selon l'invention, par exemple sous la forme d'articles textiles tels que des gazes. Il peut aussi s'agir d'une combinaison des fils, fibres, filaments ou articles textiles de l'invention avec d'autres bases textiles (par exemple, des feutres non tissés ou des mailles) ou avec des plastiques.

Le dispositif médical non implantable peut également comprendre une combinaison des fils, fibres, filaments, articles textiles et des combinaisons de ceux-ci avec d'autres bases textiles ou plastiques :
- avec d'autres fibres telles que des fibres d'alginate, de chitosane, de chitine, ou de collagène...et/ou
- avec des médicaments pour améliorer ou accélérer le processus de guérison des plaies.

L'interaction entre la composition polymérique selon l'invention et la peau améliore la cicatrisation de la peau lésée, notamment en stimulant la synthèse du collagène.

La lésion peut être de plusieurs types, il s'agit par exemple du résultat d'un traumatisme mécanique (coupure, contusion, lacération, éraflure, morsure), thermique (brûlure), électrique (électrocution) ou chimique (brûlure).

La composition selon l'invention est utile pour améliorer la cicatrisation de la peau. De manière préférée, elle est appliquée contre la partie lésée de la peau, qui est éventuellement préalablement désinfectée.

L'application recommandée pourra aller de quelques heures à plusieurs jours selon la gravité, le type et la profondeur de la lésion.

Selon l'invention, et de façon particulièrement avantageuse, la composition selon l'invention (notamment les fils, fibres, filaments, le film, l'article textile ou le dispositif médical non implantable) pourra être stérilisée avant son utilisation.

De façon surprenante, l'invention permet d'obtenir une grande efficacité dans la cicatrisation.

Les fils, fibres ou filaments, et les films obtenus à partir de la composition selon la présente invention présentent en outre l'avantage de présenter une haute résistance aux lavages/nettoyages (c'est-à-dire, une bonne rémanence de leur efficacité sur la cicatrisation) grâce à l'incorporation de des charges minérales dans la matrice polymérique.

On donne ci-après des exemples de réalisation de l'invention. Ces exemples sont donnés à titre illustratif et sans caractère limitatif.

### EXEMPLES

### 1. Réalisation de la composition polymérique

On prépare un mélange maître (masterbatch) de polyamide 66 en incorporant 20 % en poids de charges minérales émettrices d'infrarouge sous forme de poudre dans du polyamide 66 de viscosité relative (VR) 43, mesurée dans une solution d'acide formique à 90% dans l'eau.

Le mélange maître ainsi obtenu est extrudé, refroidi et granulé.

Les granulés ainsi obtenus sont refondus puis introduits lors du filage dans du polyamide 66 fondu de viscosité relative (VR) 43, mesurée dans une solution d'acide formique à 90% dans l'eau, dans une proportion permettant d'obtenir la quantité désirée de charges minérales dans la matrice polymère.

### 2. Filage du polymère et réalisation du tissu

La composition polymérique fondue obtenue est filée à une température entre 280°C et 300°C (mesurée dans la filière), refroidie à l'air (20°C, humidité relative de 65 %) et enroulée à une vitesse de 4200 m/min pour obtenir un fil continu multifilamentaire. Le fil multifilamentaire formé de 68 filaments de section circulaire a été postérieurement texturé. Le titre du filament dans le produit fini est de 1,2 dtex.

Dans l'exemple de l'invention on a réalisé un fil de polyamide 66 contenant 1,5 % en poids de TiO₂ de taille moyenne en volume de particule de 0,3 µm, 0,5 % en poids de BaSO₄ de taille moyenne en volume de particule de 0,25 µm et 0,2 % en poids de tourmaline de taille moyenne en volume de particule de 0,3 µm.

Le fil ainsi obtenu est ensuite transformé en tricots par utilisation d'une tricoteuse circulaire.

A titre comparatif, on a également réalisé un fil multifilamentaire à partir d'un polyamide 66 vierge (contenant uniquement 1,5% en poids de TiO₂ de taille moyenne en volume de particule de 0,3 µm) de viscosité relative (VR) 43, mesurée dans une solution d'acide formique à 90% dans l'eau. Le fil comparatif est également formé de 68 filaments de section circulaire et a été postérieurement texturé. Le titre du filament dans le produit fini est de 1,2 dtex.

Le fil ainsi obtenu est aussi transformé en tricots par utilisation de la même tricoteuse circulaire.

Des bermudas ont ensuite été confectionnés à partir desdits tricots. Les bermudas ont une densité de surface de 305 g/m², et contiennent 12% d'élasthanne. Ces articles ont ensuite été utilisés pour évaluer la performance dans l'application visée (cf tests in vivo ci-dessous).

### 3. Tests in vitro :

Afin d'évaluer l'effet dû à la présence du tissu selon l'invention sur la synthèse du collagène, on a utilisé une méthodologie in vitro qui a été décrite dans la littérature (Carlson, MA, Longaker, MT. Wound Repair and Regeneration,. 12(2) :134-47, 2004 Mar-Apr.) et validée par les spécialistes dans le domaine. Cette méthodologie consiste à utiliser de la matrice collagène de fibroblastes-peuplée (MCFP) comme modèle expérimental de guérison in vitro, car cela donne une estimation raisonnable de la cicatrisation de la plaie pendant les phases établies du tissu de granulation.

Selon le modèle MCFP, une solution de collagène du type I et de fibroblastes primaires est ajoutée à une culture cellulaire. Cette solution polymérise au pH physiologique et à température de 37°C donnant lieu à un gel, puis le milieu supplémenté en facteurs de croissance ou de sérum est ajouté.

Les fibroblastes ont été obtenus à partir de la peau normale de 5 volontaires. L'évaluation des échantillons de fibroblastes de chaque volontaire a été effectuée 8 fois. Les résultats donnés plus bas sont une moyenne de ces évaluations.

Les fibroblastes ont été maintenus dans des conditions définies de température et de saturation d'oxygène (37°C et 5 % de CO₂) pendant 7 jours.

Le même modèle a été construit en présence de la lumière extérieure. Après sept jours de culture, les boîtes de Petri ont été photographiées à l'aide du logiciel UTSCSA Image Tool for Windows version 3, la surface de chaque gel a été mesurée, permettant de calculer le niveau de contraction du gel.

La méthode précédemment décrite permet d'observer le niveau de contraction du gel, ce qui permet d'évaluer l'activité de synthèse du collagène par les fibroblastes. En effet, plus la contraction du gel est importante, plus la synthèse de collagène est importante.

Les résultats de contraction du gel après 7 jours sont décrits dans le tableau 1 ci-dessous

**Tableau 1: Augmentation de la contraction du gel après 7 jours.**

| Tissu utilisé | Augmentation de la Contraction (par rapport à une contraction sans tissu) |
|---|---|
| Tissu de l'exemple 1 | + 21% |
| Tissu comparatif | + 1% |

Conclusion : On constate que le taux de contraction du gel lorsqu'il est en contact avec le tissu de l'invention est nettement supérieur à celui observé avec le tissu comparatif. Ainsi, la synthèse du collagène est significativement augmentée en présence du tissu de l'invention.

### 4. Tests in vivo :

Pour évaluer la synthèse de collagène in vivo, une étude a été conduite avec un groupe de 15 femmes volontaires qui ont porté des bermudas composées avec du fil selon l'invention pour une jambe et du fil polyamide comparatif pour l'autre jambe.

Le bermuda est en contact direct avec la peau.

Après 60 jours consécutifs d'utilisation des bermudas à raison de 6 heures par jour, une évaluation de la quantité de collagène type I (le plus présent dans la peau) dans la peau a été mise en place. Les résultats donnés plus bas sont une moyenne de ces évaluations.

La peau humaine présente un niveau maximum d'excitation de la fluorescence à la longueur d'onde de 295 nm et d'émission de fluorescence à 360 nm. Cette fluorescence est attribuée à la chaîne latérale aromatique de l'aminoacide tryptophane présent dans les structures protéiques de la peau. Un autre maximum d'excitation est observé à la longueur d'onde de 340 nm et à une émission de fluorescence à 400 nm. Cet autre maximum est attribué aux liaisons croisées du collagène type I.

La méthode choisie est la mesure par spectroscopie de fluorescence qui permet de quantifier la présence de collagène type I dans la peau, en comparant la quantité de tryptophane (référence mesuré à 360 nm) avec la quantité des liaisons croisées de collagène (mesuré à 400 nm).

L'intensité du signal du tryptophane dans le spectre d'excitation à 295 nm est fortement liée à la prolifération cellulaire. Une réduction de l'intensité du signal à 295 nm indique une réduction de la prolifération épidermale associée au vieillissement de la peau.

Le vieillissement interne (intrinsèque) de la peau, présente une réduction de 10 à 20% du signal de tryptophane et de collagène type I tous les 10 ans.

En prenant en compte la courte durée du test, le signal de tryptophane a été considéré comme une référence interne pratiquement constante durant l'étude.

Le rapport entre les intensités des signaux à 340 nm et 295 nm (I₃₄₀/I₂₉₅) indique l'augmentation du nombre de liaisons croisées de collagène de type I, résultant de l'augmentation de la synthèse de cette molécule.

Les résultats de la quantification de la synthèse de collagène se trouvent dans le tableau 2 ci-dessous.

**Tableau 2: Augmentation du Collagène (%).**

| Échantillon | Augmentation après 60 jours |
|---|---|
| Tissu de l'exemple 1 | 5,9% |
| Tissu comparatif | 0,9% |

Le test statistique montre qu'il existe une différence statistiquement significative entre le tissu selon l'invention et le tissu comparatif du point de vue de l'augmentation de la synthèse de collagène in vivo. (p<0,0001).

Les deux tests in vitro et in vivo ci-dessus confirment que le contact du fil additivé selon l'invention avec la peau permet d'observer une augmentation significative de la synthèse du collagène.

Comme cela est déjà communément admis par la communauté scientifique, la synthèse de collagène joue un rôle important dans le procédé de cicatrisation de la peau et l'augmentation de sa synthèse contribue de façon significative à la qualité et à la vitesse de la cicatrisation.

## Revendications

1. Composition polymérique comprenant :
- une matrice polymérique choisie dans le groupe comprenant les polyesters, les polyoléfines, les polymères à base de cellulose-ester, les polymères et copolymères acryliques, les polyamides, leurs copolymères ou mélanges, et
- au moins trois charges minérales de types différents choisies parmi les types suivants : les oxydes, les sulfates, et les silicates, uniformément dispersées dans la matrice polymérique, ayant des propriétés d'absorption et/ou d'émission dans la région d'infrarouge lointain allant de 2 µm à 20 µm,
pour son utilisation pour améliorer la cicatrisation de la peau.

2. Composition selon la revendication 1, **caractérisée en ce que** la matrice polymérique est à base de polyamide, choisi entre le polyamide 6, le polyamide 66 et les copolymères de polyamide 6/polyamide 66 en toutes proportions.

3. Composition selon l'une quelconque des revendications précédentes, **caractérisée en ce qu'**elle contient trois charges minérales de types différents, les trois charges étant un oxyde, un sulfate, et un silicate, et de préférence l'association dioxyde de titane/sulfate de métal alcalino-terreux/silicate

4. Composition selon l'une des revendications précédentes, **caractérisée en ce que** les charges minérales se trouvent sous forme de particules présentant une taille moyenne en volume, mesurée selon la méthode d'analyse granulométrique par diffraction laser, inférieure ou égale à 2 µm, de préférence allant de 0,1 à 2 µm, et plus préférentiellement de 0,2 à 1,5 µm.

5. Composition selon l'une quelconque des revendications précédentes, **caractérisée en ce que** la proportion en poids de charges minérales par rapport au poids total de la composition polymérique est supérieure ou égale à 1,0 %, de préférence supérieure ou égale à 1,5 % et plus préférentiellement encore supérieure ou égale à 2,5 %.

6. Composition selon l'une quelconque des revendications précédentes, **caractérisée en ce que** la proportion en poids de charges minérales par rapport au poids total de la composition polymérique est inférieure ou égale à 9%, de préférence inférieure ou égale à 6 %, et plus préférentiellement encore inférieure ou égale à 4,5%.

7. Composition selon l'une quelconque des revendications précédentes, **caractérisée en ce qu'**elle se présente sous forme de fils, fibres ou filaments.

8. Composition selon la revendication 7, **caractérisée en ce que** les filaments présentent une masse linéaire allant de 0,2 à 20 dtex, de préférence de 0,5 à 8 dtex, et encore plus préférentiellement de 0,5 à 3,5 dtex.

9. Composition selon l'une quelconque des revendications 7 et 8, **caractérisée en ce que** les fils, fibres ou filaments sont contenus dans un article textile, tel que par exemple un tissu, un tricot ou un non-tissé.

10. Composition selon la revendication précédente, **caractérisée en ce que** l'article textile est un bandage ou un vêtement.

11. Composition selon l'une des revendications 1 à 6, **caractérisée en ce qu'**elle se présente sous forme de film.

12. Composition selon l'une quelconque des revendications précédentes, **caractérisée en ce qu'**elle est contenue dans un dispositif médical non implantable, tel qu'un pansement ou un fil de suture.

## Patentansprüche

1. Polymerzusammensetzung, umfassend:
- eine Polymermatrix, ausgewählt aus der Gruppe, die Polyester, Polyolefine, Polymere auf der Basis von Celluloseester, Acrylpolymere und -copolymere, Polyamide, deren Copolymere oder Gemische umfasst, und
- mindestens drei mineralische Füllstoffe unterschiedlicher Typen, die aus den folgenden Typen ausgewählt sind: Oxiden, Sulfaten und Silikaten, die in die Polymermatrix gleichmäßig dispergiert sind und Absorptions- und/oder Emissionseigenschaften im fernen Infrarotbereich von 2 µm bis 20 µm besitzen,
für ihre Verwendung zur Verbesserung der Wundheilung der Haut.

2. Zusammensetzung nach Anspruch 1, **dadurch gekennzeichnet, dass** die Polymermatrix auf Polyamid basiert, ausgewählt aus Polyamid 6, Polyamid 66 und Copolymeren von Polyamid 6/Polyamid 66 in beliebigen Verhältnissen.

3. Zusammensetzung nach einem der vorhergehenden Ansprüche, **dadurch gekennzeichnet, dass** sie drei mineralische Füllstoffe unterschiedlicher Typen enthält, wobei die drei Füllstoffe ein Oxid, ein Sulfat und ein Silikat und vorzugsweise eine Beimischung von Titandioxid/Erdalkalimetallsulfat/Silikat sind.

4. Zusammensetzung nach einem der vorhergehenden Ansprüche, **dadurch gekennzeichnet, dass** die mineralischen Füllstoffe in Form von Partikeln vorliegen, die eine gemäß dem Verfahren der Partikelgrößenanalyse mittels Laserbeugung gemessene volumengemittelte Partikelgröße von weniger als oder gleich 2 µm, vorzugsweise von 0,1 bis 2 µm und stärker bevorzugt von 0,2 bis 1,5 µm aufweisen.

5. Zusammensetzung nach einem der vorhergehenden Ansprüche, **dadurch gekennzeichnet, dass** der Gewichtsanteil an anorganischen Füllstoffen, bezogen auf das Gesamtgewicht der Polymerzusammensetzung, größer als oder gleich 1,0 %, vorzugsweise größer als oder gleich 1,5 % und noch stärker bevorzugt größer als oder gleich auf 2,5 % ist.

6. Zusammensetzung nach einem der vorhergehenden Ansprüche, **dadurch gekennzeichnet, dass** der Gewichtsanteil an anorganischen Füllstoffen, bezogen auf das Gesamtgewicht der Polymerzusammensetzung, kleiner als oder gleich 9 %, vorzugsweise kleiner als oder gleich 6 % und noch stärker bevorzugt kleiner als oder gleich 4,5 % ist.

7. Zusammensetzung nach einem der vorhergehenden Ansprüche, **dadurch gekennzeichnet, dass** sie in Form von Fäden, Fasern oder Filamenten vorliegt.

8. Zusammensetzung nach Anspruch 7, **dadurch gekennzeichnet, dass** die Filamente eine längenbezogene Masse von 0,2 bis 20 dtex, vorzugsweise von 0,5 bis 8 dtex und noch stärker bevorzugt von 0,5 bis 3,5 dtex aufweisen.

9. Zusammensetzung nach einem der Ansprüche 7 und 8, **dadurch gekennzeichnet, dass** die Fäden, Fasern oder Filamente in einen Textilgegenstand, wie zum Beispiel einem Gewebe, einem Strickstoff oder einem Vliesstoff, enthalten sind.

10. Zusammensetzung nach dem vorhergehenden Anspruch, **dadurch gekennzeichnet, dass** der Textilgegenstand eine Bandage oder ein Kleidungsstück ist.

11. Zusammensetzung nach einem der Ansprüche 1 bis 6, **dadurch gekennzeichnet, dass** sie in Filmform vorliegt.

12. Zusammensetzung nach einem der vorhergehenden Ansprüche, **dadurch gekennzeichnet, dass** sie in einer nicht-implantierbaren medizinischen Vorrichtung, wie einem Verband oder einem Nahtfaden, enthalten ist.

## Claims

1. Polymeric composition comprising:
- a polymeric matrix chosen from the group comprising polyesters, polyolefins, cellulose-ester-based polymers, acrylic polymers and copolymers, polyamides, and copolymers or mixtures thereof, and
- at least three mineral fillers of different types chosen from the following types: oxides, sulphates and silicates, uniformly dispersed in the polymeric matrix, having properties of absorption and/or emission in the far infrared region ranging from 2 µm to 20 µm
for its use for improving skin healing.

2. Composition according to Claim 1, **characterized in that** the polymeric matrix is based on polyamide, chosen from polyamide 6, polyamide 66 and copolymers of polyamide 6/polyamide 66 in any proportions.

3. Composition according to either one of the preceding claims, **characterized in that** it contains three mineral fillers of different types, the three fillers being an oxide, a sulphate and a silicate, and preferably the titanium dioxide/alkaline earth metal sulphate/silicate combination.

4. Composition according to one of the preceding claims, **characterized in that** the mineral fillers are in the form of particles having a volume average size, measured according to the method of laser diffraction particle size analysis, of less than or equal to 2 µm, preferably ranging from 0.1 to 2 µm, and more preferentially from 0.2 to 1.5 µm.

5. Composition according to one of the preceding claims, **characterized in that** the weight proportion of mineral fillers relative to the total weight of the polymeric composition is greater than or equal to 1.0%, preferably greater than or equal to 1.5% and even more preferentially greater than or equal to 2.5%.

6. Composition according to one of the preceding claims, **characterized in that** the weight proportion of mineral fillers relative to the total weight of the polymeric composition is less than or equal to 9%, preferably less than or equal to 6% and even more preferentially less than or equal to 4.5%.

7. Composition according to one of the preceding claims, **characterized in that** it is in the form of yarns, fibres or filaments.

8. Composition according to Claim 7, **characterized in that** the filaments have a linear mass ranging from 0.2 to 20 dtex, preferably from 0.5 to 8 dtex, and even more preferentially from 0.5 to 3.5 dtex.

9. Composition according to either one of Claims 7 and 8, **characterized in that** the yarns, fibres or filaments are contained in a textile article, such as, for example, a fabric, a knit or a nonwoven.

10. Composition according to the preceding claim, **characterized in that** the textile article is a bandage or a piece of clothing.

11. Composition according to one of Claims 1 to 6, **characterized in that** it is in the form of a film.

12. Composition according to any one of the preceding claims, **characterized in that** it is contained in a non-implantable medical device, such as a dressing or a suture thread.
